# EUROPÄISCHE PATENTANMELDUNG

(11) **EP 0 579 261 A1**
(43) Veröffentlichungstag der Anmeldung: **19.01.1994**
(21) Anmeldenummer: 93111849.1
(22) Anmeldetag: 05.10.1989
(51) Int. Cl.: C07C 235/60, C07C 233/54, C07C 65/05, C07C 237/44, G01N 33/84, A61K 31/165, A61K 31/19

(54) **Fluorsubstituierte Benzolderivate**

(30) Priorität: 07.10.1988 DE 3834704
(62) Teilanmeldung aus: 89250053.9
(71) Anmelder: SCHERING AKTIENGESELLSCHAFT, D-13342 Berlin (DE)
(72) Erfinder: Blaszkiewicz, Peter, Dr., D-12165 Berlin (DE); Niedballa, Ulrich, Dr., D-14195 Berlin (DE); Gries, Heinz, Dr., D-10717 Berlin (DE); Bauer, Hans, Dr., D-12107 Berlin (DE); Weinmann, Hanns-Joachim, Dr., D-14129 Berlin (DE)

(57) **Zusammenfassung**

Fluorsubstituierte Benzolderivate der allgemeinen Formel I 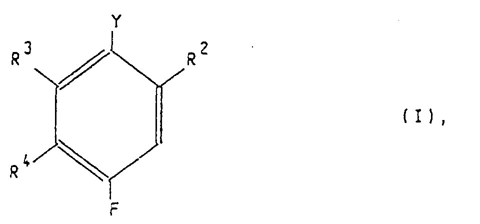
worin
Y für OH- steht und R¹, R², R³ und R⁴ unterschiedliche Bedeutung haben, sind als NMR-Diagnostika geeignet.

## Beschreibung

Die Erfindung betrifft den in den Patentansprüchen gekennzeichneten Gegenstand, das heißt Benzolderivate, die mindestens zwei Fluoratome enthalten, ihre Verwendung als NMR-Diagnostika, diagnostische Mittel, die diese Benzolderivate enthalten sowie Verfahren zur Herstellung dieser Verbindungen und Mittel.

Die moderne Medizintechnik ermöglicht es, kleinste morphologische Strukturen mit einer Auflösung darzustellen, die der von Gewebeschnitten aus Anatomielehrbüchern nahekommt.

Es gelingt jedoch auch mit Hilfe der Ultraschall-, der Röntgen-Diagnostik, der Nuklearmedizin und selbst der Kernspintomographie nicht, Informationen über den stoffwechselphysiologischen Zustand eines Gewebes des lebenden Organismus zu erhalten. Für eine genauere Diagnose und insbesondere für eine Planung und Verlaufskontrolle eines therapeutischen Einsatzes ist jedoch diese Kenntnis von erheblicher Bedeutung, da eine optimale Therapie nur dann erfolgreich sein kann, wenn frühzeitig eine Aussage über deren Wirkung möglich ist.

Ein wichtiger Parameter der stoffwechselphysiologischen Aktivität ist der pH-Wert. Viele pathologische Prozesse haben eine Veränderung der Wasserstoffionen-Konzentration zur Folge. Eines der bekanntesten Beispiele ist die Freisetzung von Milchsäure in Folge ungenügender Sauerstoffversorgung und dadurch bedingte anaerober Verstoffwechselung von Glucose. Eine anaerobe Glykolyse findet praktisch überall dort statt, wo eine ausreichende Versorgung mit Sauerstoff nicht mehr gewährleistet ist. Eine kurzfristige Ansäuerung ist zum Beispiel in Bereichen höchster Muskelaktivität festzustellen. Hier wird jedoch die anfallende Milchsäure in der Ruhepause relativ rasch abtransportiert, so daß im ruhenden Muskel keine Übersäuerung festzustellen ist. Anders sieht es jedoch in Bereichen permanenter Sauerstoffschuld aus. In ischaemischen Arealen (Infarkt) kommt es aufgrund der gesteigerten anaeroben Glykolyse zu einer Verschiebung des pH-Wertes. Ähnliche Effekte sind in schnell wachsenden Neoplasmen zu beobachten. Neben einer Regulationsstörung liegt im Bereich eines Tumors ein Sauerstoffmangel vor, so daß auch hier durch anaerobe Verstoffwechselung von Kohlehydraten eine Ansäuerung auftritt.

Die Bestimmung des Gewebe-pH-Werts führt somit zu wichtigen Aussagen über Funktion, Zustand und Wachstum der Zellen, so daß es generell wünschenswert ist, metabolische Azidosen zu lokalisieren. (Am. J. Physiol. 246, R 409, 1984; R. Nuccitelli, D.W. Deamer, Eds. 1982 Intracellular pH: Its Measurement, Regulation and Utilization in Cellular Functions, Liss. New York). Neben der Messung des pH-Wertes mit pH-Elektroden wurde neuerdings auch die NMR-Spektroskopie zu diesem Zweck eingesetzt. Mit deren Hilfe ist es erstmals gelungen, den pH-Wert des Gewebes ohne äußere Beeinflussung zu bestimmen.

Die Bestimmung des pH-Wertes mit Hilfe der NMR-Spektroskopie beruht auf der Messung der Signale einer chemischen Verbindung, die sich in einem pH-abhängigen, reversiblen Gleichgewicht befindet. Ist dieses Gleichgewicht langsam bezüglich der NMR-Zeitskala, so können die Signale sämtlicher Komponenten erhalten werden und die Signalintensitäten entsprechen den Konzentrationen der Gleichgewichtskomponenten. Bei einem schnellen Gleichgewicht ist dagegen nur ein Signal meßbar und die chemische Verschiebung ist durch die chemische Verschiebung der Gleichgewichtskomponenten und deren Konzentration gegeben.

In einem Zweikomponenten-Gleichgewicht kann dann, bei Kenntnis des pKₐ-Wertes und der chemischen Verschiebung der Komponenten, der pH-Wert mit Hilfe der Henderson-Hasselbalch-Gleichung berechnet werden.

Aus der folgenden Tabelle ist ersichtlich, welche Atomkerne prinzipiell für die NMR-Bildgebung bzw. -Spektroskopie in Frage kommen:

| Kern | Frequenz bei 1 Tesla MHz | rel. Meßem-pfindlichkeit ¹H = 1 | Konzentration in biol. Gewebe | Chemical Shift | chemische Modifikationsmöglichkeit | T₁-Relaxationszeiten |
|---|---|---|---|---|---|---|
| ¹H | 42.6 | 1,0 | 100 mol/L | klein | sehr hoch | 0.1-3 sec |
| 19_{F} | 40.1 | 0.8 | « 1 mmol/L | sehr groß | sehr hoch | 1-5 sec |
| 23_{Na} | 11.3 | 0.09 | 100 mmol/L | ./. | praktisch | < 0.1 sec |
| | | | | | null | |
| 31_{P} | 17.2 | 0.06 | 10 mmol/L | mittel | begrenzt | 1-5 sec |
| 13_{C} | 10.7 | 0.0002 | 1 mmol/L | sehr groß | sehr hoch | 1-10 sec |

Seit 15 Jahren wird der ³¹P-Kern als nicht-invasive Meßsonde für die intracelluläre pH-Wert-Messung eingesetzt (J. Biol. Chem. 248, 7276, 1973).
Das pH-empfindliche Signal ist hierbei das Signal des anorganischen Phosphats aus dem Gleichgewicht Hydrogenphosphat-Dihydrogenphosphat; als Referenz dient das ³¹P-Signal des Phosphorkreatins.
Die Verwendung des ³¹P-Kerns für die pH-Wert-Bestimmung hat jedoch auch ihre Grenzen: So ist eine exakte Bestimmung des pH-Wertes in einem gut lokalisierten Gewebevolumen beim Menschen selbst mit dem Einsatz von 2T Kernspintomographen nicht möglich. Dies liegt sowohl an den relativ niedrigen Phosphatkonzentrationen sowie an der Tatsache, daß das ³¹P-Signal meßtechnisch schwierig zu erfassen ist. Störsignale im Bereich des anorganischen Phosphats, Überlagerung des anorganischen P-Signals durch andere P-Metabolite oder das Fehlen eines Referenzsignals können eine pH-Messung verhindern. Weitere Schwierigkeiten liegen in der geringen Empfindlichkeit des Kerns und der geringen pH-Abhängigkeit der chemischen Verschiebung. Die Genauigkeit der pH-Messung wird vor allem durch die Bestimmung der chemischen Verschiebungen der Signale beeinflußt und ist nicht besser als 0,2 pH. Außerdem können Resonanzsignale bei der Verwendung endogener Phosphate gänzlich fehlen, weil die Verbindungen sich nur in so geringen Konzentrationen (z.B. im Darm oder in Ehrlich Aszites-Tumorzellen) anreichern, daß eine pH-Wert Bestimmung nicht möglich ist.
Aufgrund dieser Gegebenheiten ist nur eine recht ungenaue pH-Bestimmung in vergleichsweise großen Volumina möglich. Für die Erstellung eines befriedigenden ³¹P-Spektrums werden bei einer Akkumulationszeit von 15 Minuten Signale aus dem Meßvolumen von etwa 100 ccm aufgenommen.

Bei der Nutzung anderer Kerne als ³¹P ist der ¹⁹Fluor-Kern der Kern der Wahl, da er ein leicht meßbares NMR-Signal, das dem des Wasserstoffprotons sehr ähnlich ist (er besitzt ebenso wie ¹H einen Kernspin von 1/2), liefert, d.h. es können die gleichen Empfänger- und Sender-Spulen wie in der ¹H-NMR-Diagnostik verwendet werden, eine hohe Empfindlichkeit (ca. 83% von ¹H) besitzt, in 100% Häufigkeit vorliegt und die Signale über einen großen Frequenzbereich verteilt sind. Als weitere Vorteile sind die Abwesenheit von Fluor im Organismus (mit Ausnahme der Zähne), so daß keine Komplikationen mit endogen F-Signalen auftreten können (Fehlen eines ¹⁹F-Background-Signals) - sowie die günstige chemische Zugänglichkeit anzuführen.

Informationen, die über die Verwendung von F-Molekülen in der NMR-Diagnostik erhältlich sind, können durch kein anderes diagnostisches bildgebendes bzw. quasi-bildgebendes Verfahren erzielt werden: das Signal kann sich im Körper -je nach chemischem Zustand - stark verändern, erlaubt somit die Quantifizierung biochemischer Reaktionen und ermöglicht eine direkte Beobachtung physiologischer Vorgänge. Trotz dieser verführerischen Eigenschaften muß auf die problematische Konzentration hingewiesen werden. Ein sinnvolles Experiment bedarf ¹⁹F-Konzentrationen von > 1 mmol F/l, d.h. die zu applizierenden Verbindungen müssen eine hervorragende Verträglichkeit aufweisen und eine gute Löslichkeit in Wasser besitzen, damit durch Verwendung von Lösungen hoher Konzentrationen möglichst kleine Volumina verwendet werden können.

Die Frequenz (chemical shift) einer Fluorlinie wird durch die Lage des F-Atoms im Molekül bestimmt. Prinzipiell gilt dies für alle anderen Atomkerne auch, jedoch ist im Falle des Fluoratoms der chemical shift besonders stark ausgeprägt. Um eine Verschiebung des Fluorsignals zu beobachten bzw. zu quantifizieren bedarf es einer Bezugs(Referenz)-Linie. Diese Frequenzlinie kann das ¹H-Signal, ein externer F-Standard bzw. eine sich nicht verändernde F-Linie, die sich ebenfalls in dem zu vermessenden Areal befindet, sein. Diese Referenzlinie kann sich in einem anderen, ähnlich verteilenden Molekül oder vorzugsweise in dem als Indikator benutzten Molekül selbst befinden. Die günstigste Situation liegt im letztgenannten Fall vor, da hierbei nur eine Substanz appliziert wird und keinerlei Probleme mit Suszeptibilitätseffekten auftreten, so daß eine zweifelsfreie Zuordnung der Signale möglich ist.

Es besteht daher ein Bedarf, geeignete Verbindungen zu finden, die auf eine Veränderung des pH-Werts mit einer veränderten Meßgröße (Resonanzfrequenz) im NMR-Spektrum bei gleichzeitigem Vorhandensein einer Referenzlinie reagieren. Weiterhin müssen diese Verbindungen bzw. die diese Verbindungen enthaltenden diagnostischen Mittel folgende Eigenschaften aufweisen:
a) eine große chemische Verschiebung pro pH-Einheit;
b) geeignete pK-Werte für in-vivo-Messungen;
c) eine für die Diagnostik geeignete Pharmakokinetik:
d) eine für eine Messung genügend hohe Anreicherung in den Zielorganen;
e) gute Verträglichkeit und geringe Toxizität;
f) metabolische Stabilität;
g) hohe chemische Stabilität und Lagerfähigkeit,
h) gute Wasserlöslichkeit.

Die bisher (und nur für in-vitro-Untersuchungen!) beschriebenen Verbindungen (Annals of the New York Academy of Science, S.M. Cohen, Ed. 1987, 508 33) erfüllen diese Voraussetzungen nicht. So ist mit ihnen z.B. eine genauere pH-Bestimmung als mit ³¹P nicht möglich, da die pH-Abhängigkeit der chemischen Verschiebung zu gering ist (≦ 1 ppm/pH) und/oder ihre pH-Werte liegen außerhalb des physiologischen Bereichs und/oder ihre Resonanzfrequenzen sind nicht nur vom pH sondern auch von der Feldstärke abhängig. Auch sind die beschriebenen Verbindungen auf Grund ihrer schlechten Verträglichkeit für einen tierexperimentellen oder gar klinischen Einsatz nicht geeignet.

Der Erfindung liegt somit die Aufgabe zugrunde, Verbindungen und Mittel die die oben genannten Eigenschaften aufweisen zur Verfügung zu stellen, sowie Verfahren zu ihrer Herstellung zu schaffen. Diese Aufgabe wird durch die Erfindung gelöst.

Es wurde gefunden, daß sich fluorsubstituierte Benzolderivate der allgemeinen Formel I 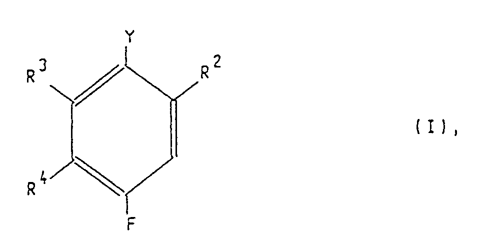
worin
- Y: eine OH-Gruppe
- R²: Wasserstoff, Fluor, eine 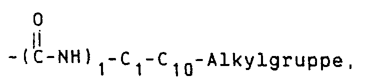 die gerad- oder verzweigtkettig und gegebenenfalls mit 1 bis 6 Fluoratomen substituiert ist, wobei 1 für die Ziffern 0 oder 1 steht,
den Rest N(R⁶)-CO-R⁸
worin R⁸ einen gerad- oder verzweigtkettigen Alkylrest mit 1 bis 16 Kohlenstoffatomen, der gegebenenfalls durch 1 bis 6 Hydroxygruppen und 1 bis 12 Fluoratome substituiert und gegebenenfalls durch 1 bis 3 Sauerstoffatome unterbrochen ist, bedeutet und
R⁶ Wasserstoff, eine gerad- oder verzweigtkettige, gegebenenfalls durch 1 bis 6 Hydroxygruppen oder 1 bis 12 Fluoratome substituierte Alkylgruppe mit 1 bis 16 Kohlenstoffatomen
oder einen Rest 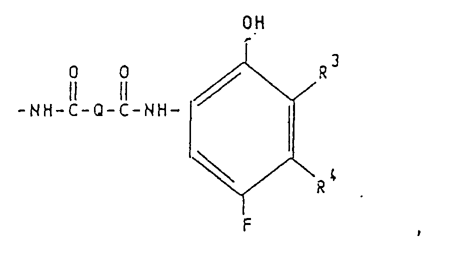 worin Q für eine (CF₂)ₖ- oder C₆F₄-Gruppe mit k in der Bedeutung der Ziffern 1, 2, 3, 4, 5 oder 6 steht,
- R³ und R⁴: unabhängig voneinander Wasserstoff, Fluor, eine gerad- oder verzweigtkettige Alkylgruppe mit 1 bis 10 Kohlenstoffatomen, die gegebenenfalls durch 1 bis 6 Fluoratome substituiert ist, den Rest -N(R⁶)-CO-R⁸ oder den Rest V bedeuten,
worin V für Wasserstoff oder die Reste U-OR⁵ oder 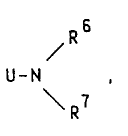 wobei
U einen CO- oder SO₂-Rest,
R⁵ Wasserstoff, einen gesättigten, ungesättigten, gerad- oder verzweigtkettigen oder cyclischen, gegebenenfalls durch 1 bis 6 Hydroxygruppen substituierten Kohlenwasserstoffrest mit bis zu 16 Kohlenstoffatomen und
R⁷ die für R⁶ angegebene Bedeutung hat oder gemeinsam mit R⁶ unter Einbeziehung des Stickstoffatoms einen gegebenenfalls ein weiteres Heteroatom enthaltenden gesättigten Fünf- oder Sechsring bilden,
mit der Maßgabe, daß mindestens 2 Fluoratome im Molekül vorhanden sind, die im Molekül vorhandenen Reste V gleich oder verschieden sein können, und gewünschtenfalls freie Säuregruppen mit organischen oder anorganischen Basen versalzt sind,
überraschenderweise hervorragend zur Herstellung von NMR-Diagnostika eignen.

Stehen R¹ und X¹ , X² bzw. R², R³ und R⁴ für eine Alkylen- bzw. Alkylgruppe, so kann diese gerad- oder verzweigtkettig sein, bis zu 10 Kohlenstoffatome aufweisen und gegebenenfalls durch 1 bis 6 Fluoratome substituiert sein. Bevorzugt sind Alkylen- bzw. Alkylsubstituenten mit 1 bis 6 Kohlenstoffatomen, die gegebenenfalls durch 1 bis 6 Fluoratome substituiert sind. Beispielsmäßig genannt seien Methylen, Difluormethylen, Ethylen, Methylethylen, Ethylethylen, Trimethylen, Tetramethylen, Pentamethylen, Hexamethylen, 1,1-Difluorethylen, 1-Fluorethylen, 1-Methyltetramethylen, 1-Methyltrimethylen, 2-Methyltrimethylen, 2-Methyltetramethylen, Trifluormethylmethylen, 2,2-Difluorethylen bzw. im Falle von R², R³ und R⁴ die entsprechenden um ein Wasserstoff- oder Fluoratom ergänzten Alkylreste wie zum Beispiel Methyl, Fluormethyl, Trifluormethyl, Ethyl, 2-Fluorethyl, 2,2,2-Trifluorethyl, 1-Methylethyl, 1-Fluormethylethyl, 2-Fluor-1-methylethyl usw.

Als Substituenten R⁵, R⁶, R⁷ und R⁸ können gesättigte, ungesättigte, gerad-oder verzweigtkettige oder cyclische, vorzugsweise gesättigte Kohlenwasserstoffreste mit bis zu 16 Kohlenstoffatomen, die gegebenenfalls durch 1 bis 6 Hydroxygruppen sowie im Falle von R⁶, R⁷ und R⁸ durch 1 bis 12 Fluoratome substituiert sind, in Betracht.
Beispielsmäßig genannt seien die Methyl-, Ethyl-, Hydroxymethyl-, 1-Hydroxyethyl-, 2-Hydroxyethyl-, 2-Hydroxy-1-(hydroxymethyl)ethyl-, 1-(Hydroxymethyl)ethyl-, Propyl-, Isopropyl-, Isopropenyl-, 2- und 3-Hydroxypropyl-, 2,3-Dihydroxypropyl-, Butyl, Isobutyl-, Isobutenyl-, 2-, 3-und 4-Hydroxybutyl-, 2-, 3- und 4-Hydroxy-2-methylbutyl-, 2- und 3-Hydroxy-isobutyl-, 2,3,4Trihydroxybutyl-, Cyclohexyl-, Pentyl-, Hexyl-, Bis-und Tris(hydroxymethyl)methyl-, 2,3-Dihydroxy-1-(hydroxymethyl)propyl-, 2,3,4,5,6-Pentahydroxyhexyl, 1,3,4-trihydroxybutyl-2-, sowie 2,2,2-Trifluorethyl-, Trifluormethyl-, 2Fluorethyl-, 1-Fluormethylethylgruppe.
Bevorzugt sind Kohlenwasserstoffreste mit bis zu 6 Kohlenstoffatomen und gegebenenfalls 1 bis 5 Hydroxygruppen sowie 1 bis 8 Fluoratome.
Im Falle von R⁸ kann der Alkylrest durch 1 bis 3 Sauerstoffatome unterbrochen sein. Als Beispiele für in R⁸ enthaltene Kettenglieder seien aufgeführt:
-CH₂-O-CH₂-, -CH₂-CH₂-O-CH₂-CH₂-, -(CH₂-O-CH₂)₂-, -(CH₂-O-CH₂)₃-, -CH₂-CH₂-(OCH₂-CH₂)₃-.

Wenn R⁶ und R⁷ gemeinsam mit dem Stickstoffatom einen gegebenenfalls ein weiteres Heteroatom enthaltenden gesättigten Fünf- oder Sechsring darstellen, steht 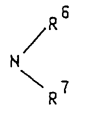
vorzugsweise für Pyrrolidin, Piperidin, Morpholin oder Piperazin.

Die in den Verbindungen der allgemeinen Formel vorhandenen aciden Wasserstoffatome können gegebenenfalls ganz oder teilweise durch Kationen anorganischer und/oder organischer Basen oder Aminosäuren ersetzt sein. Geeignete anorganische Kationen sind beispielsweise das Lithiumion, das Kaliumion, das Calciumion das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die Herstellung der fluorsubstituierten Benzolderivate der allgemeinen Formel I erfolgt dadurch, daß man
in Verbindungen der allgemeinen der allgemeinen Formel II 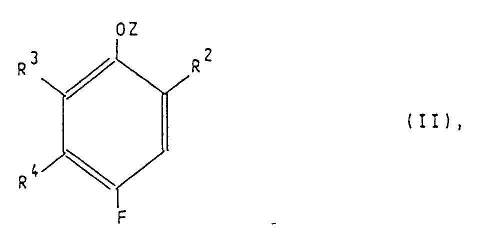
worin
Z für eine Hydroxyschutzgruppe steht, die Schutzgruppe Z abspaltet und gegebenenfalls in R³ und R⁴ vorhandene Schutzgruppen entfernt und gewünschtenfalls vorhandene freie Säuregruppen mit organischen oder anorganischen Basen versalzt.

Als Schutzgruppe Z kommen alle Hydroxylschutzgruppen infrage, die bekanntermaßen für einen intermediären Hydroxylgruppenschutz geeignet sind, d.h. die sich leicht einführen und sich später unter Rückbildung der letztlich gewünschten freien Hydroxylgruppe auch wieder leicht abspalten lassen. Bevorzugte Schutzgruppen sind Ethergruppen wie z.B. Benzyl-, Di-und Triphenyl-methyl-Ethergruppen sowie Acetal- und Ketalgruppen mit z.B. Acetaldehyd und Aceton. Geeignet ist auch oft der Schutz durch Veresterung z.B. durch Einführung des Benzoyl- oder Acyl-, insbesondere des Acetylrestes.

Die Abspaltung der Schutzgruppen Z erfolgt - ebenso wie die der in R³ und R⁴ gegebenenfalls vorhandenen Schutzgruppen - in an sich bekannter Weise, z.B. im Falle eines Benzylethers durch hydrogenolytische Spaltung in Gegenwart von z.B. Palladium-Kohle, im Falle eines Esters z.B. durch alkalische Verseifung in wäßrig-alkoholischer Lösung bei Temperaturen von 0 bis 50°C bzw. bei tert-Butylestern mit Hilfe von Trifluoressigsäure, sowie im Falle einer Ketalspaltung mit Hilfe von z.B. Kationenaustauschern oder Trifluoressigsäure.

Die gegebenenfalls durchzuführenden Folgereaktionen zu den Endsubstanzen mit den gewünschten Substituenten R³ und R⁴, wie z.B. Verseifung von Estern, Überführung einer Carbon- oder Sulfonsäure in ein Amid sowie Abspaltung von Schutzgruppen folgt literaturbekannten Methoden. Sollen Polyhydroxyalkylamide hergestellt werden, so ist es vorteilhaft die dafür benötigten Polyhydroxyalkylamine in geschützter Form in die Reaktion einzusetzen, z.B. als O-Acylderivate oder als Ketale. Dies gilt besonders dann, wenn diese Derivate bequemer und billiger herstellbar sind als die Polyhydroxyalkylamine selbst. Ein typisches Beispiel ist das 2-Amino-1-(2,2-dimethyl-1,3-dioxolan-4-yl)-ethanol, das Acetonid des 1-Amino-2,3,4-trihydroxybutans, hergestellt nach DE-OS 31 50 917.

Die nachträgliche Entfernung der Schutzgruppen ist problemlos und kann z. B. durch Behandlung mit einem sauren Ionenaustauscher in wäßrig-ethanolischer Lösung erfolgen.

Die für die oben angeführte Reaktion benötigten Edukte der allgemeinen Formel II sind käuflich oder literaturbekannt oder können analog literaturbekannten Methoden hergestellt werden (z.B. J. Org. Chem. 27, 3134 (1962), J. of Organometallic Chem. 190 [1980], 217, J. Org. Chem. 1984, 49, 2792, JACS 73 [1951], 1325, J. Org. Chem. 1982, 47, 1081, Tet. Lett. 1984, 839).

Nach Herstellung der gewünschten Verbindung der allgemeinen Formel I können im Molekül vorhandene acide Wasserstoffatome durch Kationen anorganischer und/oder organischer Basen oder Aminosäuren substituiert werden.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium und Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren.
Zur Herstellung der neutralen Salze kann man beispielsweise den sauren Benzolderivaten in wäßriger Lösung oder Suspension ein Äquivalent der gewünschten Basen zusetzen. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol. Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten.
Enthalten die sauren Verbindungen mehrere freie acide Gruppen, so ist es oft zweckmäßig, neutrale Mischsalze herzustellen, die sowohl anorganische als auch organische Kationen als Gegenionen enthalten.

Die Herstellung der erfindungsgemäßen diagnostischen Mittel erfolgt ebenfalls in an sich bekannter Weise, indem man die erfindungsgemäßen Verbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Komplexbildnern (wie zum Beispiel Diethylentriaminpentaessigsäure) oder, falls erforderlich, Elektrolyte wie zum Beispiel Natriumchlorid oder, falls erforderlich, Antioxidantien wie zum Beispiel Ascorbinsäure.
Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween^{(R)}, Myrj^{(R)} und/oder Aromastoff(en) zur Geschmackskorrektur (zum Beispiel ätherischen Ölen) gemischt.

Die erfindungsgemäßen fluorhaltigen Verbindungen können vorteilhaft in der in-vivo NMR-Diagnostik, d.h. für die NMR-Bildgebung und in der NMR-Spektroskopie als Indikator verschiedener Parameter eingesetzt werden. So können unter anderem mit Hilfe der ortsaufgelösten Spektroskopie und damit gewebsspezifisch pH, pO₂, pCO₂, Temperatur, Redox-Vorgänge, Reaktionskinetik gemessen werden.
Weiterhin wurde festgestellt, daß sich die erfindungsgemäßen Verbindungen überraschenderweise durch eine sehr gute Verträglichkeit auszeichnen. So wurde zum Beispiel für 5-[N-(4-Flour-2-trifluormethylphenyl)sulfamoyl]-isophthalsäure (Beispiel 13) an der Maus eine intravenöse akute Verträglichkeit (LD₅₀) von 4 mmol/kg Körpergewicht ermittelt. Dieser Befund ist überraschend, da die meisten vergleichbaren fluorierten Verbindungen eine äußerst schlechte (<0.1 mmol/kg Körpergewicht) Verträglichkeit aufweisen.

Die erfindungsgemäßen pharmazeutischen Mittel werden vorzugsweise in einer Konzentration von 1 µMol-1 Mol/l hergestellt. Sie werden in der Regel in Mengen von 0,005-20 mMol/kg Körpergewicht, vorzugsweise 0,05 bis 5 mMol/kg Körpergewicht, dosiert. Sie sind zur enteralen und parenteralen Applikation bestimmt.

Die erfindungsgemäßen Mittel erfüllen die vielfältigen Voraussetzungen für die Eignung als Diagnostika für die NMR-Tomographie und -Spektroskopie. Ferner zeigen sie die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit der erfindungsgemäßen Mittel erlaubt es, hochkonzentrierte Lösungen herzustellen, damit die Volumenbelastung des Kreislaufs in vertretbaren Grenzen zu halten und die Verdünnung durch Körperflüssigkeit auszugleichen, das heißt NMR-Diagnostika müssen 100-1000-fach besser wasserlöslich sein als die in der in-vitro NMR-Spektroskopie verwendeten Mittel.

Die gute Verträglichkeit der erfindungsgemäßen Verbindungen erlaubt die Prüfung und NMR-spektroskopische Vermessung des pH-Wertes im lebenden Organismus. Hierbei ermöglicht eine Gabe von 10 µmol/kg bis 10 mmol/kg Körpergewicht eine problemlose Bestimmung der Veränderung der chemischen Verschiebung des ¹⁹F-Signals im Verhältnis zum Referenzsignal (z.B. einer intramolekularen CF₃-Gruppe) und damit des pH-Wertes. Die applizierte Lösung verteilt sich rasch im Organismus und ist somit in der Lage, Bereiche unterschiedlichen pH-Wertes nachzuweisen. Durch eine entsprechende Dosierung kann darüberhinaus eine Veränderung des pH-Wertes und damit gegebenenfalls ein therapeutischer Effekt bewirkt werden.

Um kleine Änderungen des pH-Wertes aufzeigen zu können, sind die Verbindungen von Vorteil, deren pK-Wert in der Nähe des biologischen bzw. pathologischen pH-Wertes des interessierenden Gewebes ist. In der Regel sind diejenigen Verbindungen von besonderem Interesse, deren pK-Wert zwischen 2 und 9, vorzugsweise zwischen 6 und 8, liegt. Verbindungen, die den pH-Wert des Magen-Darm-Traktes aufzeigen, haben vorteilhafterweise einen pK zwischen 2 und 8. Da die größte Genauigkeit der pH-Bestimmung im Bereich der größten Veränderung der chemischen Verschiebung pro Einheit, also beim pK-Wert der jeweiligen Verbindung, vorliegt ist eine sehr gute Analyse biologischer Vorgänge möglich. So liegt der pH des Blutes bei etwa 7.2-7.4; pathologische Bereiche können einen veränderten pH-Wert haben, der z.B. bis auf 4.0 oder niedriger sinken kann.

Für die Darstellung der Nierenfunktion bzw. Analyse des Primär- und Sekundärharnes sind Verbindungen mit einem pK-Wert zwischen 5 und 7 von Vorteil, da der pH-Wert des Urins in der Regel unter dem des Blutes liegt. Für die Bestimmung des intragastralen pH-Wertes sind die Verbindungen von Vorteil, die eine Veränderung der chemischen Verschiebung zwischen pH 2 und 6 am deutlichsten zeigen, da der pH-Wert des Magensaftes zwischen fast 1 und 7 stark schwanken kann.

Durch die Verwendung der sehr gut verträglichen neuartigen fluorierten Meßsonden ist es somit möglich geworden, in kleineren Volumina (z.B. 10 ccm) ortsaufgelöste Spektroskopie durchzuführen und physiologisch wichtige Parameter wie z.B. den pH-Wert präzise in kurzer Meßzeit ohne Störung bzw. Überlagerung durch andere Moleküle zu bestimmen.

Für ein in-vivo Imaging (NMR-Bildgebung) sind die genannten Verbindungen ebenfalls geeignet. Hierbei werden nicht nur die Informationen der veränderten chemischen Verschiebung bildlich dargestellt, sondern es werden die lokalen Konzentrationen der fluorierten Verbindungen durch die in der MRT üblichen Aufnahmesequenzen in einem Imaging wiedergegeben. Der Vorteil des ¹⁹F-Imaging gegenüber der ¹H-Tomographie beruht darin, daß die Verteilung des Pharmakons direkt ohne Überlagerung durch störende Strukturen dargestellt werden kann.

So gelingt z.B. eine hervorragende Kontrastierung des renalen Ausscheidungssystems (Niere, Urether, Blase) nach intravenöser Gabe der erfindungsgemäßen Verbindungen, welche in einer Dosis van 5 µmol/kg bis 20 mmol/kg, vorzugsweise von 0,1 mmol/kg bis 5 mmol/kg, appliziert werden. Hierbei wurde überraschenderweise auch gefunden, daß die zusätzliche Injektion einer paramagnetischen Verbindung (z.B. GdDTPA/Dimeglumin) in einer Dosis von 1 µmol/kg bis 2 mmol/kg, vorzugsweise von 50 µmol/kg bis 500 µmol/kg, zu einer deutlichen Verbesserung der Bildqualität führt.

Gekoppelt an zum Beispiel geeignete monoklonale Antikörper können die erfindungsgemäßen Verbindungen auch Verwendung als organ- und tumorspezifische Therapeutika und Diagnostika finden.

Die nachfolgenden Beispiele dienen zur näheren Erläuterung des Erfindungsgegenstandes.

### Beispiel 1

### 5-Flour-2-hydroxy-3-(2,2,2-trifluorethylcarbamoyl)-benzoesäure

### a) (2,6-Dibrom-4-flor-phenyl)-benzylether

2.21g (96 mmol) Natrium werden unter Feuchtigkeitsausschluß in 66ml Ethanol gelöst. Zu dieser Lösung fügt man 21,6g (80 mmol) 2,6-Dibrom-4-fluorphenol hinzu. Es entsteht eine Suspension. Dieser werden 20.5g (120 mmol) Benzylbromid zugesetzt, 30 Minuten bei Raumtemperatur gerührt und anschließend 1 Stunde am Rück fluß gekocht. Eine Probe der Suspension reagiert dann auf feuchtem Indikatorpapier neutral. Der Niederschlag des Natriumbromids wird abgesaugt, das Filtrat zur Trockne eingedampft und der Rückstand aus Ethanol kristallisiert. Man erhält 23.7g (66 mmol) = 82.5% d. Th. Kristallisat. Fp. 83°C.

### b) 2-Benzyloxy-5-fluor-isophthalsäure-diethylester

10.8g (30 mmol) (2,6-Dibrom-4-fluor-phenyl)-benzylether werden in einer Argonatmospäre unter Feuchtigkeitsausschluß in 150ml trockenem Tetrahydrofuran gelöst und die Lösung auf -100°C gekühlt. Bei dieser Temperatur werden 225ml (180 mmol) einer 0.8-molaren Lösung von Butyllithium in Hexan zugetropft. Nach beendeter Zugabe rührt man 5 Minuten nach und tropft dann eine Lösung von 21.24g (180 mmol) Diethylcarbonat in 50ml trockenem Tetrahydrofuran ein. Die Temperatur darf dabei -40°C nicht übersteigen. Anschließend rührt man nach, bis das Reaktionsgemisch Raumtemperatur erreicht hat und weitere 2 Stunden bei dieser Temperatur. Das Reaktionsgemisch wird unter vermindertem Druck eingedampft, der Rückstand in Essigester gelöst und die Lösung mehrmals mit Wasser ausgeschüttelt. Die organische Phase wird über Natriumsulfat getrocknet, im Vakuum eingedampft und der Rückstand an Kieselgel 60 (Merck) mit Essigester/Hexan chromatografiert. Man erhält 5.43g (15.69 mmol) 52.3% d. Th des Diesters als amorpen Feststoff.

| Analyse : C₁₉H₁₉FO₅ MG 346.35 | | | | | | | | |
|---|---|---|---|---|---|---|---|---|
| berechnet: | C | 65.99 | H | 5.53 | F | 5.48 | O | 23.09% |
| gefunden: | | 66.17 | | 5.77 | | 5.26 | | % |

### c) 2-Benzyloxy-5-flour-isophthalsäure-monoethylester

25.63g (74 mmol) 2-Benzyloxy-5-fluor-isophthalsäure-diethylester werden unter Feuchtigkeitsausschluß in 200ml trockenem Ethanol gelöst und in diese Lösung im Verlaufe von 2 Stunden bei Raumtemperatur eine Lösung von 1.48g Natriumhydroxyd in 50ml Ethanol eingetropft. Das Mono-Natriumsalz fällt allmählich aus. Nach beendetem Eintropfen wird 1 Stunde nachgerührt, der Niederschlag abgesaugt und das Filtrat auf ca. die Hälfte eingeengt. Dabei fällt weiteres Mono-Natriumsalz, das ebenfalls abgesaugt wird. Beide Filterrückstände werden vereinigt, in Wasser suspendiert und die Suspension mit konzentrierter Salzsäure angesäuert. Die Monosäure wird mit Essigester ausgeschüttelt, die Essigesterlösung über Natriumsulfat getrocknet, filtriert und eingedampft. Der feste Rückstand wird im Vakuum bei 50°C getrocknet. Man erhält 14.93g (46.92 mmol) = 63.4% d. Th. als teils kristallinen, teils amorphen Feststoff.

| Analyse : C₁₇H₁₅FO₅ MG 318.3 | | | | | | | | |
|---|---|---|---|---|---|---|---|---|
| berechnet: | C | 64.14 | H | 4.75 | F | 5.96 | O | 25.13% |
| gefunden : | | 64.35 | | 4.51 | | 6.13 | | % |

### d) 2-Benzyloxy-5-fluor-3-(2,2,2-trifluorethylcarbamoyl)-benzoesäure-ethylester

7.96g (25 mmol) 2-Benzyloxy-5-fluor-isophthalsäure-monoethylester werden in 50ml trockenem Essigester gelöst, bzw. suspendiert, 3.57g (30 mmol) Thionylchlorid hinzugefügt und 1 Stunde bei 50°C gerührt. Die Reaktionslösung wird dann unter vermindertem Druck zur Trockne eingedampft, der Rückstand zweimal mit je 50ml Dichlormethan abgedampft und in 30ml trockenem Tetrahydrofuran gelöst. Diese Lösung wird mit 3.03g (30 mmol) Triethylamin versetzt und anschließend eine Lösung von 2.97g (30 mmol) 2,2,2-Trifluorethylamin in 10ml trockenem Tetrahydrofuran eingetropft. Man läßt 5 Stunden bei Raumtemperatur nachrühren, dampft die Reaktionslösung unter vermindertem Druck zum Öl ein, löst
dieses in 80ml Essigester und schüttelt zweimal mit je 10ml 1N Salzsäure und einmal mit 10ml Wasser aus. Die organische Phase wird über Magnesiumsulfat getrocknet, filtriert, eingedampft und der Rückstand an Kieselgel 60 (Merck) mit Essigester/Hexan chromatografiert. Man erhält 7.25g (18.15 mmol)= 72.6% d. Th. der Verbindung als amorphen Feststoff.

| Analyse : C₁₉H₁₇F₄NO₄ MG 399.34 | | | | | | | | | | |
|---|---|---|---|---|---|---|---|---|---|---|
| berechnet: | C | 57.14 | H | 4.29 | F | 19.03 | N | 3.5 | O | 16.02% |
| gefunden : | | 57.37 | | 4.53 | | 18.87 | | 3.61 | | % |

### e) 2-Benzyloxy-5-fluor-3-(2,2,2-trifluorethylcarbamoyl)-benzoesäure

10.78g (27 mmol) 2-Benzyloxy-5-fluor-3-(2,2,2-trifluorethylcarbamoyl)-benzoesäureethylester werden in 60ml Dioxan gelöst, die Lösung mit 17.5ml (35 mmol) 2N Natronlauge versetzt, 1 Stunde bei Raumtemperatur und 1 Stunde bei 40°C gerührt. Das Reaktionsgemisch wird unter vermindertem Druck eingedampft, der Rückstand in 80ml Wasser aufgenommen, mit konzentrierter Salzsäure auf pH 1 angesäuert. Die Säure fällt dabei größtenteils als amorpher Feststoff aus. Er wird abgesaugt und mit wenig Wasser gewaschen. Die wässrige Phase wird mehrfach mit Essigester ausgeschüttelt. Der Essigesterextrakt wird eingedampft und der Rückstand mit der Fällung aus Wasser vereinigt. Man trocknet 48 Stunden bei 50°C im Vakuum. Ausbeute: 8.38g (22.57 mmol) = 83.6% d. Th. als amorpher Feststoff.

| Analyse : C₁₇H₁₃F₄NO₄ MG 371.29 | | | | | | | | | | |
|---|---|---|---|---|---|---|---|---|---|---|
| berechnet: | C | 54.99 | H | 3.52 | F | 20.46 | N | 3.77 | O | 17.23% |
| gefunden : | | 55.22 | | 3.78 | | 20.27 | | 3.58 | | % |

### f) 5-Fluor-2-hydroxy-3-(2,2,2-trifluorethylcarbamoyl)-benzoesäure

5.2g (14 mmol) 2-Benzyloxy-5-fluor-3-(2,2,2-trifluorethylcarbamoyl)-benzoesäure werden in 30ml Ethanol gelöst, 250mg Palladium-Kohle (10%-ig) hinzugefügt und 1 Stunde bei Normaltemperatur hydriert. Der Katalysator wird anschließend abfiltriert und das Filtrat im Vakuum zur Trockne eingedampft. Der Rückstand wird an Kieselgel 60 (Merck) mit Dichormethan/Methanol chromatografiert. Man erhält 3.03g (11.34 mmol) = 81% d. Th. der Verbindung als amorphen Feststoff.

| Analyse : C₁₀H₇F₄O₄ MG 267.16 | | | | | | | | |
|---|---|---|---|---|---|---|---|---|
| berechnet: | C | 44.95 | H | 2.64 | F | 28.44 | O | 23.95% |
| gefunden : | | 44.76 | | 2.83 | | 28.21 | | % |

### Beispiel 2

### 5-Fluor-2-hydroxy-3-(2,2,2-trifluorethylcarbamoyl)-benzoesäure-(2,3,4-trihydroxy-butyl)-amid

### a) 2-Benzyloxy-5-fluor-3-(2,2,2-trifluorethylcarbamoyl)-benzoesäure-[2-hydroxy-2-(2,2-dimethyl-1,3-dioxolan-4-yl)-ethylamid]

4.46g (12 mmol) 2-Benzyloxy-5-fluor-3-(2,2,2-trifluorethylcarbamoyl)-benzoesäure werden unter Feuchtigkeitsausschluß in 30ml trockenem N,N-Dimethylformamid gelöst, 1.85g (12 mmol) Hydroxybenztriazol, 2.43g (15 mmol) 2-Hydroxy-2-(2,2-dimethyl-1,3-dioxolan-4-yl)-ethylamin hinzugefügt und die Lösung auf -10°C gekühlt. Zur gekühlten Lösung werden protionsweise 2.68g (13 mmol) Dicyclohexylcarbodiimid hinzugegeben, 1 Stunde bel -10°C und 6 Stunden bei Raumtemperatur gerührt. Der Niederschlag des Dicyclohexylharnstoffs wird dann abgesaugt, das Filtrat im Vakuum zur Trockne eingedampft und der Rückstand in ca. 100ml Essigester gelöst. Die Lösung wird mit gesättigter Bicarbonatlösung und mit Wasser ausgeschüttelt, über Natriumsulfat getrocknet, filtriert und eingeengt und an Kieselgel 60 (Merck) mit Essigester/Hexan chromatografiert. Man erhält 4.22g (8.2 mmol) = 68.3% d. Th. der Verbindung als amorphen Feststoff.

| Analyse : C₂₄H₂₆F₄N₂O₆ MG 514.47 | | | | | | | | | | |
|---|---|---|---|---|---|---|---|---|---|---|
| berechnet: | C | 56.03 | H | 5.09 | F | 14.77 | N | 5.44 | O | 18.65% |
| gefunden : | | 55.81 | | 4.9 | | 14.92 | | 5.23. | | % |

### b) 5-Fluor-2-hydroxy-3-(2,2,2-trifluorethylcarbamoyl)-benzoesäure-(2,3,4-trihydroxybutyl)-amid

6.95g (13.5 mmol) 2-Benzyloxy-5-fluor-3-(2,2,2-trifluorethylcarbamoyl)-benzoesäure-[2-hydroxy-2-(2,2-dimethyl-1,3-dioxolan-4-yl)-ethylamid] werden in 20ml Methanol gelöst, 5ml Wasser und 5ml 1N Salzsäure hinzugefugt und 3 Stunden bei Raumtemperatur gerührt. Die Lösung wird dann durch Zugabe von Anionenaustauscher Amberlite IRA 67 auf pH 6.3 gestellt, 200mg Palladium-Kohle (10%-ig) hinzugefügt und 1 Stunde bei Normaldruck hydriert. Der Katalysator wird anschließend abfiltriert, das Filtrat im Vakuum zur Trockne eingedampft und der Rückstand an Kieselgel 60 (Merck) mit Dichlormethan/Methanol chromatografiert. Man erhält 3.76g (9.77 mmol) = 72.4% d. Th. der Verbindung als amorphen Feststoff.

| Analyse : C₁₄H₁₆F₄N₂O₆ MG 384.28 | | | | | | | | | | |
|---|---|---|---|---|---|---|---|---|---|---|
| berechnet: | C | 43.75 | H | 4.19 | F | 19.77 | N | 7.29 | O | 24.98% |
| gefunden : | | 43.96 | | 4.32 | | 19.58 | | 7.13 | | % |

### Beispiel 3

### 5-Fluor-2-hydroxy-3-(2,2,2-trifluorethylcarbamoyl)-benzoesäure-N-methyl-N-(2,3,4-trihydroxy-butyl)-amid

### a) 2-Benzyloxy-5-fluor-3-(2,2,2-trifluorethylcarbamoyl)-benzoesäure-N-methyl-N-[2-hydroxy-2-(2,2-dimethyl-1,3-dioxolan-4-yl)-ethylamid]

2.97g (8 mmol) 2-Benzyloxy-5-fluor-3-(2,2,2-trifluorethylcarbamoyl)-benzoesäure werden unter Feuchtigkeitsausschluß in 20ml trockenem N,N-Dimethylformamid gelöst, 1.08g (8 mmol) Hydroxybenztriazol, 1.76g (10 mmol) N- Methyl-[2-Hydroxy-2-(2,2-dimethyl-1,3-dioxolan-4-yl)]- ethylamin hinzugefügt und die Lösung auf -10°C gekühlt. Zur gekühlten Lösung werden protionsweise 1.85g (9 mmol) Dicyclohexylcarbodiimid hinzugegeben, 1 Stunde bei -10°C und 5 Stunden bei Raumtemperatur gerührt. Der Niederschlag des Dicyclohexylharnstoffs wird dann abgesaugt, das Filtrat im Vakuum zur Trockne eingedampft und der Rückstand in ca. 100ml Essigester gelöst. Die Lösung wird mit gesättigter Bicarbonatlösung und mit Wasser ausgeschüttelt, über Natriumsulfat getrocknet, filtriert, eingeengt und an Kieselgel 60 (Merck) mit Essigester/Hexan chromatografiert. Man erhält 2.69g (5.09 mmol) = 63.6% d. Th. der Verbindung als amorphen Feststoff

| Analyse : C₂₅H₂₈F₄N₂O₆ MG 528.5 | | | | | | | | | | |
|---|---|---|---|---|---|---|---|---|---|---|
| berechnet: | C | 56.81 | H | 5.34 | F | 14.37 | N | 5.3 | O | 18.16% |
| gefunden : | | 56.64 | | 5.47 | | 14.51 | | 5.43 | | % |

### b) 5-Fluor-2-hydroxy-3-(2,2,2-trifluorethylcarbamoyl)-benzoesäure-N-methyl-N-(2,3,4-trihydroxy-butyl)-amid

3.86g (7.3 mmol) 2-Benzyloxy-5-fluor-3-(2,2,2-trifluorethylcarbamoyl)-benzoesäure-N-methyl-N-[2-hydroxy-2-(2,2-dimethyl-1,3-dioxolan-4-yl)-ethylamid] werden in 10ml Methanol gelöst, 10ml Wasser und 200mg Kationenaustauscher Amberlyst 15 hinzugefügt und 2 Stunden am Rückfluß gekocht. Der lonenaustauscher wird dann abfiltriert, dem Filtrat 150mg Palladium-Kohle (10%-ig) zugefügt und 1 Stunde bei Normaldruck hydriert. Der Katalysator wird danach abfiltriert, das Filtrat im Vakuum zur Trockne eingedampft und der Rückstand an Kieselgel 60 (Merck) mit Dichlormethan/Methanol chromatografiert. Man erhält 2.16g (5.42 mmol) = 74.3% d. Th. der Verbindung als amorphen Feststoff.

| Analyse : C₁₅H₁₈F₄N₂O₆ MG 398.31 | | | | | | | | | | |
|---|---|---|---|---|---|---|---|---|---|---|
| berechnet: | C | 45.23 | H | 4.55 | F | 19.07 | N | 7.03 | O | 24.1% |
| gefunden : | | 45.47 | | 4.4 | | 18.91 | | 6.87 | | % |

### Beispiel 4

### 5-Fluor-2-hydroxy-3-(trifluormethyl)-benzoesäure

### a) 2-Brom-4-fluor-6-(trifluormethyl)-phenol

9g (50 mmol) 4-Fluor-2-(trifluormethyl)-phenol werden in 100ml Dichlorethan gelöst, die Lösung bei 50°C gerührt und eine Lösung von 9.6g (60 mmol) Brom in 30ml Dichlorethan im Verlaufe von 2 Stunden zugetropft. Nach beendetem Zutropfen wird 1 Stunde bei 50°C nachgerührt und dann im Vakuum eingedampft. Der ölige Rückstand wird in 100ml Dichlorethan gelöst und dreimal mit je 20ml Wasser ausgeschüttelt. Die organische Phase wird über Magnesiumsulfat getrocknet, filtriert, eingedampft und der Rückstand an Kieselgel 60 mit Essigester/Hexan chromatografiert. Man erhält 8.07g (31.15 mmol) = 62.3% d. Th. als teilweise kristallinen Feststoff.

| Analyse : C₇H₃BrF₄O MG 259.0 | | | | | | | | | | |
|---|---|---|---|---|---|---|---|---|---|---|
| berechnet: | C | 32.46 | H | 1.16 | Br | 30.85 | F | 29.34 | O | 6.17% |
| gefunden : | | 32.23 | | 1.05 | | 31.03 | | 29.11 | | % |

### b) [2-Brom-4-Fluor-6-(trifluormethyl)-phenyl]-benzylether

0.65g (28 mmol) Natrium werden in 35ml trockenem Ethanol gelöst, 5.96g (23 mmol) 2-Brom-4-fluor-6-(trifluormethyl)-phenol hinzugefügt, 20 Minuten bei Raumtemperatur gerührt, 7.17g (42 mmol) Benzylbromid zugegeben und 1 Stunde am Rückfluß gekocht. Aus der Lösung fällt Natriumbromid kristallin aus. Die warme Lösung wird filtriert, das Filtrat zur Trockne eingedampft und der Rückstand an Kieselgel 60 mit Dichlormethan/Hexan chromatografiert. Man erhält 6.29g (18 mmol) = 78.3% d. Th. als überwiegend kristallinen Feststoff.

| Analyse : C₁₄H₉BrF₄O MG 349.13 | | | | | | | | | | |
|---|---|---|---|---|---|---|---|---|---|---|
| berechnet: | C | 48.16 | H | 2.59 | Br | 22.88 | F | 21.76 | O | 4.58% |
| gefunden : | | 48.33 | | 2.73 | | 22.67 | | 21.59 | | % |

### c) 2-Benzyloxy-5-fluor-3-(trifluormethyl)-benzoesäureethyleste

6.11g (17.5 mmol) [2-Brom-4-Fluor-6-(trifluormethyl)-phenyl]-benzylether werden in einer Argonatmosphäre unter Feuchtigkeitsausschluß in 60ml trockenem Tetrahydrofuran gelöst und die Lösung auf -100°C gekühlt. Bei dieser Temperatur werden 65.63ml (52.5 mmol) einer 0.8-molaren Lösung von Butyllithium in Hexan zugetropft. Nach beendeter Zugabe rührt man 5 Minuten nach und tropft dann eine Lösung von 3.54g (30 mmol) Diethylcarbonat in 20ml trockenem Tetrahydrofuran ein. Die Temperatur wird dabei unter -40°C gehalten. Anschließend rührt man nach, bis das Reaktionsgemisch Raumtemperatur erreicht hat und weitere 3 Stunden bei dieser Temperatur. Das Reaktionsgemisch wird unter vermindertem Druck engedampft, der Rückstand in Essigester gelöst und die Lösung mehrmals mit Wasser ausgeschüttelt. Die organische Phase wird über Magnesiumsulfat getrocknet, im Vakuum eingedampft und der Rückstand an Kieselgel 60 (Merck) mit Essigester/Hexan chromatografiert. Man erhält 5.43g (10.26 mmol) 58.6% d. Th. der Verbindung als amorpen Feststoff.

| Analyse : C₁₇H₁₄F₄O₃ MG 342.29 | | | | | | | | |
|---|---|---|---|---|---|---|---|---|
| berechnet: | C | 59.65 | H | 4.12 | F | 22.2 | O | 14.02% |
| gefunden : | | 59.83 | | 4.27 | | 21.93 | | % |

### d) 5-Fluor-2-hydroxy-3-(trifluormethyl)-benzoesäure

4.28g (12.3 mmol) 2-Benzyloxy-5-fluor-3(trifluormethyl)-benzoesäureethylester werden in 20ml Dioxan gelöst, 7.5ml (15 mmol) 2N Natronlauge hinzugefügt und 1 Stunde bei 50°C gerührt. Die Reaktionslösung wird dann mit 200mg Palladium-Kohle (10%-ig) versetzt und 1 Stunde bei Normaltemperatur hydriert. Der Katalysator wird anschließend abfiltriert, das alkalische Filtrat mit 30ml Wasser versetzt und mit Dichlormethan extrahiert. Die Wasserphase wird mit konzentrierter Salzsäure auf pH1 angesäuert und mit Essigester extrahiert. Der Essigesterextrakt wird über Magnesiumsulfat getrocknet, filtriert und zur Trockne eingedampft. Der kristalline Rückstand wird im Vakuum bei 50°C 24 Stunden getrocknet. Man erhält 2.27g (10.15 mmol) 82.5% d. Th. Schmelzbereich 76-83°C.

| Analyse : C₈H₄F₄O₃ MG 224.11 | | | | | | | | |
|---|---|---|---|---|---|---|---|---|
| berechnet: | C | 42.87 | H | 1.79 | F | 33.9 | O | 21.41% |
| gefunden : | | 42.66 | | 1.93 | | 33.76 | | % |

### Beispiel 5

### 5-Fluor-2-hydroxy-3-(trifluormethyl)-benzoesäure-(2,3,4-trihydroxy-butyl)-amid

### a) 2-Benzyloxy-5-fluor-(trifluormethyl)-benzoesäure-[2-hydroxy-2-(2,2-dimethyl-1,3-dioxolan-4-yl)-ethylamid]

3.42g (10 mmol) 2-Benzyloxy-5-fluor-3-(trifluormethyl)-benzoesäureethylester werden in 10ml Ethanol gelöst, 2.43g (15 mmol) 2-Hydroxy-2-(2,2-dimethyl-1,3-dioxolan-4-yl)-ethylamin hinzugefügt, das Ethanol abdestilliert und das zurückbleibende Öl 2 Stunden im Vakuum auf 100°C erwärmt. Das Reaktionsgemisch wird dann In 50ml Essigester gelöst, zweimal mit je 10ml 1N Salzsäure ausgeschüttelt, die organische Phase über Natriumsulfat getrocknet, eingedampft und der Rückstand an Kieselgel 60 mit Essigester/Hexan chromatografiert. Man erhält 2.18g (7.18 mmol) = 47.6.8% d. Th. als amorphen Feststoff.

| Analyse : C₂₂H₂₃F₄NO₅ MG 457.42 | | | | | | | | | | |
|---|---|---|---|---|---|---|---|---|---|---|
| berechnet: | C | 57.76 | H | 5.06 | F | 16.61 | N | 3.06 | O | 17.48% |
| gefunden : | | 57.92 | | 5.23 | | 16.45 | | 2.87 | | % |

### b) 5-Fluor-2-hydroxy-3-(trifluormethyl)-benzoesäure-(2,3,4-trihydroxy-butyl)-amid

6.86g (15 mmol) 5-Fluor-2-hydroxy-3-(trifluormethyl)-benzoesäure-[2-hydroxy-2-(2,2-dimethyl-1,3-dioxolan-4-yl)-ethylamid] werden in 30ml Methanol gelöst, 30ml Wasser und 300mg Kationenaustauscher Amberlyst 15 hinzugefügt und 2 Stunden am Rückfluß gekocht. Der Ionenaustauscher wird dann abfiltriert, dem Filtrat 250mg Palladium-Kohle (10%-ig) zugesstzt und 1 Stunde bei Normaldruck hydriert. Der Katalysator wird danach abfiltriert, das Filtrat im Vakuum zur Trockne eingedampft und der Rückstand an Kieselgel 60 (Merck) mit Dichlormethan/Methanol chromatografiert. Man erhält 3.08g (9.41 mmol) = 62.7% d. Th. der Verbindung als amorphen Feststoff.

| Analyse : C₁₂H₁₃F₄NO₅ MG 327.23 | | | | | | | | | | |
|---|---|---|---|---|---|---|---|---|---|---|
| berechnet: | C | 44.04 | H | 4.0 | F | 23.22 | H | 4.28 | O | 24.44% |
| gefunden: | | 43.88 | | 4.16 | | 22.95 | | 4.43 | | % |

### Beispiel 6

### 5-Fluor-2-hydroxy-3-(trifluormethyl)-benzoesäure-N-methyl-N-(2,3,4-trihydroxy-butyl)-amid

### a) 2-Benzyloxy-5-fluor-3-(trifluormethyl)-benzoesäure-N-methyl-N-[2-hydroxy-2(2,2-dimethyl-1,3-dioxolan-4-yl)-ethyl]-amid

4.66g (13.6 mmol) 2-Benzyloxy-5-fluor-3-(trifluormethyl)-benzoesäureethylester werden in 15ml Ethanol gelöst, 3.59g (20.4 mmol) N-Methyl-N-[2-hydroxy-2-(2,2-dimethyl-1,3-dioxolan-4-yl)]-ethylamin hinzugefügt, das Ethanol abdestilliert und das zurückbleibende Öl 2 Stunden im Vakuum auf 100°C erwärmt. Das Reaktionsgemisch wird dann in 80ml Essigester gelöst, zweimal mit je 10ml 1N Salzsäure ausgeschüttelt, die organische Phase über Magnesiumsulfat getrocknet, eingedampft und der Rückstand an Kieselgel 60 mit Essigester/Hexan chromatografiert. Man erhält 3.31g (7.03 mmol) = 51.7% d. Th. als amorphen Feststoff.

| Analyse : C₂₃H₂₅F₄NO₅ MG 471.45 | | | | | | | | | | |
|---|---|---|---|---|---|---|---|---|---|---|
| berechnet: | C | 58.59 | H | 5.34 | F | 16.11 | N | 2.97 | O | 16.96% |
| gefunden : | | 58.47 | | 5.51 | | 15.92 | | 3.11 | | % |

### b) 5-Fluor-2-hydroxy-3-(trifluormethyl)-benzoesäure-N-methyl-N-(2,3,4-trihydroxybutyl)-amid

8.01g (17 mmol) 5-Fluor-2-hydroxy-3-(trifluormethyl)-benzoesäure-N-methyl-N-[2-hydroxy-2-(2,2-dimethyl-1,3-dioxolan-4-yl)-ethyl]-amid werden in 35ml Methanol gelöst, 35ml Wasser und 350 mg Kationenaustauscher Amberlyst 15 hinzugefügt und 2 Stunden am Rückfluß gekocht. Der lonenaustauscher wird dann abfiltriert, dem Filtrat 350mg Palladium-Kohle (10%-ig) zugesetzt und 1 Stunde bei Normaldruck hydriert. Der Katalysator wird anschließend ahfiltriert, das Filtrat im Vakuum zur Trockne eingedampft und der Rückstand an Kieselgel 60 (Merck) mit Dichlormethan/Methanol chromatografiert. Man erhält 3.9g (11.44 mmol) = 67.3% d. Th. der Verbindung als amorphen Feststoff.

| Analyse : C₁₃H₁₅F₄NO₅ MG 341.26 | | | | | | | | | | |
|---|---|---|---|---|---|---|---|---|---|---|
| berechnet: | C | 45.75 | H | 4.43 | F | 22.26 | N | 4.1 | O | 23.44% |
| gefunden : | | 46.03 | | 4.57 | | 21.98 | | 4.22 | | % |

### Beispiel 7

### Hexafluorglutarsäure-bis[(5-fluor-2-hydroxy-3-hydroxycarbonyl)-anilid]

### a) Hexafluorglutarsäure-bis[(2-hydroxy-3-hydroxycarbonyl-5-nitro)-anilid]

9.91g (50 mmol) 3-Amino-2-hydroxy-5-nitro-benzoesäure (J. Chem. Soc.111, 540 [1917]) werden in 30ml trockenem Dimethylformamid gelöst, 10.12g (0.1mol) Triethylamin hinzugefügt, die Lösung auf 0°C gekühlt und bei dieser Temperatur 6.9g (25 mmol) Hexafluorglutarsäuredichlorid eingetropft. Es wird 30 Minuten bei 0°C und 3 Stunden bei Raumtemperatur nachgerührt. Der Niederschlag von Triethylaminhydrochlorid wird abgesaugt und das Filtrat in 300ml Dichlormethan gefällt. Die Fällung wird abgesaugt, in Methanol gelöst und an Kieselgel 60 mit Dichlormethan/Methanol chromatografiert. Man erhält 13.09g (21.8 mmol) = 43.6% d. Th. der Verbindung als teils amorphen, teils kristallinen Feststoff.

| Analyse : C₁₉H₁₀F₆N₄O₁₂ MG 600.3 | | | | | | | | | |
|---|---|---|---|---|---|---|---|---|---|
| berechnet: | C | 38.01 H | 1.67 | F | 18.98 | N | 9.98 | O | 31.98% |
| gefunden: | | 37.8 | 1.83 | | 19.2 | | 9.75 | | % |

### b) Hexafluorglutarsäure-bis[(2-hydroxy-3-ethoxycarbonyl-5-nitro)-anilld]

12g (20 mmol) Hexafluorglutarsäure-bis[(2-hydroxy-3-hydroxycarbonyl-5-nitro)-anilid] werden in 120ml trockenem Ethanol gelöst, 2ml konzentrierte Schwefelsäure hinzugefügt und die Lösung 6 Stunden am Rückfluß gekocht. Die Schwefelsäure wird durch Zugabe von festem Natriumhydrogencarbonat abgepuffert, bis die überstehende Lösung auf wasserfeuchtem pH-Papier pH 8 anzeigt. Der Feststoff wird abgesaugt und das Filtrat zur Trockne eingedampft. Der Rückstand wird an Kiesegel 60 mit Hexan/Essigester chromatografiert. Man erhält 7.04g (10.72 mmol) = 53.6% d. Th. als amorphen Feststoff.

| Analyse : C₂₃H₁₈F₆N₄O₁₂ MG 656.4 | | | | | | | | | | |
|---|---|---|---|---|---|---|---|---|---|---|
| berechnet: | C | 42.08 | H | 2.76 | F | 17.36 | N | 8.53 | O | 29.24% |
| gefunden: | | 42.25 | | 2.93 | | 17.13 | | 8.38 | | % |

### c) Hexafluorglutarsäure-bis[(2-benzyloxy-3-ethoxycarbonyl-5-nitro)-anilid]

1.5g (65 mmol) Natrium werden in einer Stickstoffatmosphäre unter Feuchtigkeitsausschluß in 100ml trockenem Ethanol gelöst. Zu dieser Lösung werden 17.7g (27 mmol) Hexafluorglutarsäure-bis[(2-hydroxy-3-ethoxycarbonyl-5-nitro)-anilid] hinzugefügt 15 Minuten bei Raumtemperatur gerührt, 11.12g (65 mmol) Benzylbrornid hinzugefügt und 4 Stunden am Rückfluß gekocht. Die Reaktionslosung wird dann zur Trockne eingedampft, der Rückstand in Essigester aufgenommen, filtriert und das Filtrat an Kieselgel 60 mit Hexan/Essigester chrornatografiert. Man erhält 10.73g (12.83 mmol) = 47.5% d. Th. der Verbindung als amorphen Feststoff.

| Analyse : C₃₇H₃₀F₆N₄O₁₂ MG 836.65 | | | | | | | | | | |
|---|---|---|---|---|---|---|---|---|---|---|
| berechnet: | C | 53.11 | H | 3.61 | F | 13.62 | N | 6.69 | O | 22.94% |
| gefunden : | | 53.32 | | 3.77 | | 13.48 | | 6.5 | | % |

### d) Hexafluorglutarsäure-bis[(5-amino-2-benzyloxy-3-ethoxycarbonyl)anilid]

8.37g (10 mmol) Hexafluorglutarsäure-bis[(2-benzyloxy-3-ethoxycarbonyl-5-nitro)-anilld] werden in 84ml Ethanol gelöst, 11.3g (50 mmol) Zinn-II-chlorid * H₂O hinzugefügt und die Suspension bei 70°C Badtemperatur 4 Stunden gerührt. Das Reaktionsgemisch wird zu einem Öl eingedampft und dieses an Kieselgel 60 mit Dichlormethan/Essigester chromatografiert. Man erhält 5.64g (7.26 mmol) = 72.6% der Verbindung als amorphen Feststoff.

| Analyse : C₃₇H₃₄F₆N₄O₈ MG 776.69 | | | | | | | | | | |
|---|---|---|---|---|---|---|---|---|---|---|
| berechnet: | C | 57.21 | H | 4.41 | F | 14.67 | N | 7.21 | O | 16.47% |
| gefunden: | | 57.43 | | 4.6 | | 14.43 | | 6.96 | | % |

### e) Hexafluorglutarsäure-bis[(2-benzyloxy-3-ethoxycarbonyl-5-fluor)-anilid]

9.32g (12 mmol) Hexafluorglutarsäure-bis[(5-amino-2-]benzyloxy-3-ethoxycarbonyl)-anilid] werden in einem Gemisch aus 9.6ml (60 mmol) 42%-iger Tetrafluorborsäure und 10ml Wasser suspendiert. Zu dieser Suspension tropft man bei 10-15°C Innentemperatur eine Lösung von 2.07g (30 mmol) Natriumnitrit in 10ml Wasser. Man rührt 30 Minuten bei 0-5°C nach, saugt den Niederschlag ab, wäscht ihn mit 20ml kalter 5%-iger Tetrafluorborsäure, 20ml kaltem Ethanol und 50ml kaltem Ether. Das Salz wird dann in 100ml m-Xylol suspendiert und die Suspension im Verlaufe von ca. 2 Stunden allmählich zum Sieden erhitzt. Das Xylol wird unter vermindertem Druck abdestilliert. Der Rückstand wird in 50ml Ethanol aufgenommen, 1.5ml konzentrierte Schwefelsäure zugegeben und 3 Stunden am Rückfluß gekocht. Man kühlt auf Raumtemperatur, fügt Natriumhydrogencarbonat hinzu, bis der Überstand neutrales pH anzeigt und filtriert den Niederschlag ab. Das Filtrat wird weitgehend eingedampft und der Rückstand an Kieselgel 60 mit Dichlormethan/Essigester chromatografiert. Man erhält 3.34g (4.27 mmol) = 35.6% d. Th. der Verbindung als amorphen Feststoff.

| Analyse : C₃₇H₃₀F₈N₂O₈ MG 782.64 | | | | | | | | | | |
|---|---|---|---|---|---|---|---|---|---|---|
| berechnet: | C | 56.78 | H | 3.86 | F | 19.42 | N | 3.57 | O | 16.35% |
| gefunden: | | 56.62 | | 4.03 | | 19.23 | | 3.43 | | % |

### f) Hexafluorglutarsäure-bis[(5-fluor-2-hydroxy-3-hydroxycarbonyl)-anilid]

5.87g (7.5 mmol) Hexafluorglutarsäure-bis[(2-benzyloxy-3-ethoxycarbonyl-5-fluor) -anilid] werden in 25ml Dioxan gelöst, 150mg Pd-Kohle (10%-ig) hinzugefügt und 2 Stunden bei Normaldruck hydriert. Der Katalysator wird abgesaugt und das Filtrat mit 15ml 2N Natronlauge 2 Stunden bei 50°C gerührt. Die Lösung wird mit konzentrierter Salzsäure auf ca. pH 8 gestellt und im Vakuum weitgehend eingedampft. Der ölige Rückstand wird in Wasser gelöst und auf pH 1 angesäuert. Das Produkt fällt größtenteils als amorpher Niederschlag aus. Die Wasserphase wird mit Chloroform ausgeschüttelt. Niederschlag und Chloroformextrakt werden vereinigt, eingedampft und der Rückstand an Kieselgel 60 mit Dichlormethan/Aceton chromatografiert. Man erhält (4.76 mmol) = 63.4% d. Th. als amorphen Feststoff.

| Analyse : C₁₉H₁₀F₈N₂O₈ MG 546.28 | | | | | | | | | | |
|---|---|---|---|---|---|---|---|---|---|---|
| berechnet: | C | 41.77 | H | 1.84 | F | 27.82 | N | 5.12 | O | 23.43% |
| gefunden: | | 41.96 | | 2.04 | | 27.66 | | 4.93 | | % |

### Beispiel 8

### Hexafluorglutarsäure-bis{[5-fluor-2-hydroxy-3-(2,3,4-trihydroxybutylcarbamoyl)]-anilid}

5.0g (6.4 mmol) Hexafluorglutarsäure-bis[(5-amino-2-benzyloxy-3-ethoxycarbonyl)-anilid] werden in 15ml Ethanol gelöst, 3.24 g (20 mmol) 2-Hydroxy-2-(2,2-dimethyl-1,3-dioxolan-4-yl)-ethylamin hinzugefügt, das Ethanol abdestilliert und das verbleibende Öl 3 Stunden im Vakuum bei 100-110°C erwärmt. Das Reaktionsgemisch wird anschließend auf Raumtemperatur gekühlt, in Aceton aufgenommen und an Kieseigel 60 mit Hexan/Aceton chromatografiert. Die zusammengefaßten Fraktionen des intermediären Produktes mit den Benzyl- und Ketalschutzgruppen werden eingedampft und der Rückstand in 50 Methanol gelöst. Dieser Lösung werden 200mg Pd-Kohle (10%-ig) zugefügt und 2 Stunden bei Normaldruck hydriert. Der Katalsator wird abfiltriert, dem Filtrat 30ml Wasser und 500mg Kationenaustauscher Amberlyst 15 zugesetzt und 3 Stunden bei 60°C gerührt. Der Ionenaustauscher wird dann abfiltriert, das Filtrat im Vakuum zur Trockne eingedampft und der Rückstand an Kieselgel mit Dichlormethan/Methanol chromatografiert. Man erhält 2g (2.66 mmol) = 41.5% d. Th. als amorphen Feststoff.

| Analyse : C₂₇H₂₈F₈N₄O₁₂ MG 752.52 | | | | | | | | | | |
|---|---|---|---|---|---|---|---|---|---|---|
| berechnet: | C | 43.29 | H | 3.75 | F | 20.19 | N | 7.44 | O | 25.51% |
| gefunden : | | 43.42 | | 3.9 | | 19.93 | | 7.27 | | % |

### Beispiel 9

### 5-Fluor-3-hydroxyacetamido-2-hydroxy-benzoesäure-(2,2,2-trifluor-ethyl)amid

### a) 5-Fluor-3-nitro-salicylsäure

6,24 g (40 mmol) 5-Flour-salicylsäure werden unter Erwärmen in 62,4 ml Acetonitril gelöst, die Lösung auf 0 °C gekühlt und unter Beibehaltung dieser Temperatur eine Lösung von 2,96 ml (44 mmol) 65 %iger HNO₃ in 14,8 ml Acetonitril zugetropft. Das Reaktionsgemisch wird 24 Stunden bei 0 °C gerührt, dann über 7,5 g Kieselgel 60 filtriert und zur Trockne eingedampft. Man erhält 6,1 g = 75,8 % d. Th. Rohprodukt, das ohne Reinigung in die nächste Reaktion eingesetzt wird.

### b) 5-Fluor-2-hydroxy-3-nitro-benzoesäure-(2,2,2-trifluor-ethyl)amid

5,4 g (26,85 mmol) 5-Fluor-2-hydroxy-3-nitro-benzoesäure werden in 27 ml Thionylchlorid suspendiert und bei 50 °C gerührt. Es entsteht eine nahezu klare Lösung. Nach 1 Stunde wird die Lösung im Vakuum zur Trockne eingedampft, der Rückstand in 27 ml THF gelöst und 27 ml 2,2,2-Trifluorethylamin hinzugefügt. Die Lösung wird 15 Stunden bei Raumtemperatur gerührt, dann Filtriert, das Filtrat eingedampft und an Kieselgel 60 mit Methylenchlorid/Essigester chromatographiert. Man erhält 3,51 g = 46,33 % d. Th. als amorphen Feststoff. Dieser ist aus Dioxan kristallisierbar.

| Analyse: C₉H₆F₄N₂O₄ MG 282,15 | | | | | | | | | | |
|---|---|---|---|---|---|---|---|---|---|---|
| berechnet: | C | 38,31 | H | 2,14 | F | 26,93 | N | 9,92 | O | 22,68 % |
| gefunden: | | 38,05 | | 2,32 | | 26,65 | | 9,63 | | |

### c) 3-Amino-5-fluor-2-hydroxy-benzoesäure-(2,2,2-frifluor-ethyl)amid

7,05 g (25 mmol) 5-Fluor-2-hydroxy-3-nitro-benzoesäure-(2,2,2-trifluorethyl)amid werden in 70 ml Ethanol gelöst, 5,65 (25 mmol) SnCl₂.H₂O hinzugefügt und die Suspension 2 Stunden bei 70 °C gerührt. Das Reaktionsgemisch wird dann zu einem Öl eingedampft und ohne Reinigung in die nächste Reaktion eingesetzt.

### d) 3-Acetoxyacetamido-5-fluor-2-hydroxy-benzoesäure-(2,2,2-trifluorethyl)amid

6,3 g (25 mmol) rohes 3-Amino-5-fluor-2-hydroxy-benzoesäure-(2,2,2-trifluor-ethyl)amid werden in 30 ml THF gelöst, 3,04 g (30 mmol) Triethylamin sowie 4,1 g (30 mmol) Acetoxyacetylchlorid hinzugefügt und 12 Stunden bei Raumtemperatur gerührt. Die Reaktianslösung wird dann im Vakuum eingedampft und der Rückstand an Kieselgel 60 mit Essigester/Hexan chromatographiert. Man erhält 5,52 g = 62,7 % d. Th. als amorphen Feststoff.

| Analyse: C₁₃H₁₂F₄N₂O₅ MG 352,24 | | | | | | | | | | |
|---|---|---|---|---|---|---|---|---|---|---|
| berechnet: | C | 44,32 | H | 3,43 | F | 21,57 | N | 7,95 | O | 22,71 % |
| gefunden: | | 44,67 | | 3,52 | | 21,31 | | 7,78 | | |

### e) 5-Fluor-3-hydroxyacetamido-2-hydroxy-benzoesäure-(2,2,2-trifluor-ethyl)amid

5,9 g (17 mmol) 3-Acetoxyacetamido-5-fluor-2-hydroxy-benzoesäure-(2,2,2-trifluor-ethyl)amid werden in 34 ml 1N NaOH gelöst und 30 Minuten bei 40 °C gerührt. Die Reaktionslösung wird dann auf Raumtemperatur gekühlt und über 200 ml Kationenaustauscher filtriert. Das Eluat wird im Vakuum zur Trockne eingedampft und der Rückstand an Kieselgel 60 mit Essigester/Aceton chromatographiert. Man erhält 3,3 g = 62,7 % d. Th. als amorphes Material.

| Analyse: C₁₁H₁₀F₄N₂O₄ | | | | | | | | | | |
|---|---|---|---|---|---|---|---|---|---|---|
| berechnet: | C | 42,59 | H | 3,24 | F | 24,49 | N | 9,03 | O | 20,63 % |
| gefunden: | | 42,83 | | 3,52 | | 24,21 | | 8,87 | | |

### Beispiel 10

### 5-Fluor-3-[N-(2,3-dihydroxy-propyl)-hydroxyacetamido-2-hydroxy-benzoesäure-(2,2,2-trifluor-ethyl)amid

### a) 5-Fluor-3-acetoxyacetamido-2-benzyloxy-benzoesäure-(2,2,2-trifluor-ethyl)-amid

7,75 g (22 mmol) 3-Acetoxyacetamido-5-fluor-2-hydroxy-benzoesäure-(2,2,2-trifluor-ethyl)amid [Beispiel 37d)] werden in 50 ml trockenem THF gelöst, 1,35 g (25 mmol) Natriummethylat hinzugefügt, 15 Minuten bei Raumtemperatur gerührt, 4,27 g (25 mmol) Benzylbromid zugefügt und 1 Stunde am Rückfluß gekocht. Die Reaktionslösung wird dann auf Raumtemperatur gekühlt, NaBr abfiltriert, das Filtrat im Vakuum eingedampft und der Rückstand an Kieselgel 60 mit Essigester/Hexan chromatographiert. Man erhält 5,2 g = 53,4 % d. Th. als amorphen Feststoff.

| Analyse: C₂₀H₁₈F₄N₂O₅ MG 442,37 | | | | | | | | | | |
|---|---|---|---|---|---|---|---|---|---|---|
| berechnet: | C | 54,3 | H | 4,1 | F | 17,17 | N | 6,33 | O | 18,08 % |
| gefunden: | | 54,57 | | 4,32 | | 16,88 | | 6,17 | | |

### b) 5-Fluor-3-[N-(2,2-dimethyl-1,3-dioxolan-4-ylmethyl)-acetoxyacetamido-2-benzyloxy-benzoesäure-(2,2,2-trifluor-ethyl)amid

6,64 g (15 mmol) 5-Fluor-3-acetoxyacetamido-2-benzyloxy-benzoesäure-(2,2,2-trifluor-ethyl)amid werden in 50 ml THF gelöst, 480 mg (20 mmol) NaH hinzugefügt, 30 Minuten bei Raumtemperatur gerührt, 3,01 g (20 mmol) 4-Chlormethyl-2,2-dimethyl-dioxolan hinzugefügt und 3 Stunden am Rückfluß gekocht. Die Reaktionslösung wird dann im Vakuum eingedampft und der Rückstand an Kieselgel 60 mit Essigester/Hexan chromatographiert. Man erhält 4,03 g = 48,3 % d. Th. als amorphen Feststoff.

| Analyse: C₂₆H₂₈F₄N₂O₇ MG 556,51 | | | | | | | | | | |
|---|---|---|---|---|---|---|---|---|---|---|
| berechnet: | C | 56,11 | H | 5,07 | F | 13,65 | N | 5,07 | O | 20,12 % |
| gefunden: | | 56,32 | | 5,32 | | 13,44 | | 4,93 | | |

### c) 5-Fluor-3-[N-(2,3-dihydroxy-propyl)-hydroxyacetamido-2-hydroxy-benzoesäure-(2,2,2-trifluor-ethyl)amid

6,68 g (12 mmol) 5-Fluor-3-[N-(2,2-dimethyl-1,3-dioxolan-4-ylmethyl)-acetoxyacetamido-2-benzyloxy-benzoesäure-(2,2,2-trifluor-ethyl)amid werden in 40 ml Methanol gelöst, 15 ml 1N methanolische NaOH zugefügt und 30 Minuten bei 40 °C gerührt. Die Lösung wird dann mit 40 ml Ionenaustauscher Amberlyst 15 3 Stunden bei Raumtemperatur gerührt, filtriert, das Filtrat im Vakuum eingedampft, der Rückstand in 40 ml Methanol gelöst, 200 mg Palladium-Kohle (10 %ig) zugefügt und 1 Stunde bei Normaldruck hydriert. Der Katalysator wird anschließend abgesaugt, das Filtrat zur Trockne eingedampft und der Rückstand an Kieselgel 60 mit Methylenchlorid/Methanol chromatographiert. Man erhält 2,6 g = 56,6 % d. Th. als amorphen Feststoff.

| Analyse: C₁₄H₁₆F₄H₂O₆ MG 384,28 | | | | | | | | | | |
|---|---|---|---|---|---|---|---|---|---|---|
| berechnet: | C | 43,75 | H | 4,19 | F | 19,77 | N | 7,29 | O | 24,98 % |
| gefunden: | | 43,92 | | 4,37 | | 19,53 | | 7,02 | | |

### Beispiel 11

### 5-Fluor-3-(D-gluconamido)-2-hydroxy-benzoesäure-(2,2,2-trifluorethyl)amid

### a) 5-Fluor-3-(pentaacetoxy-D-gluconamido)-2-hydroxy-benzoesäure-(2,2.2-trifluor-ethyl)amid

4,29 g (17 mmol) 3-Amino-5-fluor-2-hydroxy-benzoesäure-(2,2,2-trifluorethyl)amid [Beispiel 37c)] werden in 40 ml THF gelöst, 2,03 g (20 mmol) Triethylamin und 8,5 g (20 mmol) Pentaacetoxy-D-gluconsäurechlorid hinzugefügt und 12 Stunden bei Raumtemperatur gerührt. Das Reaktionsgemisch wird dann im Vakuum eingedampft, der Rückstand in Essigester gelöst und mit Wasser ausgeschüttelt. Die organische Phase wird über MgSO₄ getrocknet, filtriert, eingedampft und der Rückstand an Kieselgel 60 mit Essigester/Hexan chromatographiert. Man erhält 68, 2 % d. Th. als amorphen Feststoff.

| Analyse: C₂₅H₂₈F₄N₂O₈ MG 560,5 | | | | | | | | | | |
|---|---|---|---|---|---|---|---|---|---|---|
| berechnet: | C | 53,37 | H | 5,03 | F | 13,55 | N | 4,99 | O | 22,83 % |
| gefunden: | | 53,58 | | 5,26 | | 13,37 | | 4,72 | | |

### b) 5-Fluor-3-(gluconamido)-2-hydroxy-benzoesäure-(2,2,2-trifluor-ethyl)amid

(8,5 mmol) 5-Fluor-3-(pentaacetoxy-D-gluconamido)-2-hydroxy-benzoesäure-(2,2,2-trifluor-ethyl)amid werden in 30 ml Methanol gelöst, 10 ml 1N methanolische NaOH zugefügt und 1 Stunde bei 40 °C gerührt. Dann werden 30 ml Kationenaustauscher Amberlyst 15 zugefügt und 1 Stunde bei Raumtemperatur gerührt. Der Ionenaustauscher wird abfiltriert, das Filtrat zur Trockne eingedampft, der Rückstand mehrfach mit Methylenchlorid extrahiert und anschließend im Vakuum bei 50 °C getrocknet. Man erhält 76,3 % d. Th. als amorphen Feststoff.

| Analyse: C₁₅H₁₈F₄N₂O₈ MG 430,31 | | | | | | | | | | |
|---|---|---|---|---|---|---|---|---|---|---|
| berechnet: | C | 41,86 | H | 4,21 | F | 17,66 | N | 6,51 | O | 29,74 % |
| gefunden: | | 42,07 | | 4,42 | | 17,49 | | 5,32 | | |

## Patentansprüche

1. Fluorsubstituierte Benzolderivate der allgemeinen Formel I 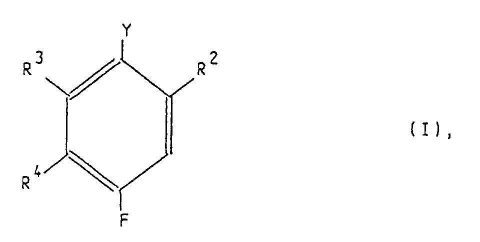 worin
Y eine OH-Gruppe
R² Wasserstoff, Fluor, eine 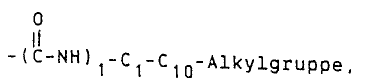 die gerad- oder verzweigtkettig und gegebenenfalls mit 1 bis 6 Fluoratomen substituiert ist, wobei l für die Ziffern 0 oder 1 steht,
den Rest N(R⁶)-CO-R⁸
worin R⁸ einen gerad- oder verzweigtkettigen Alkylrest mit 1 bis 16 Kohlenstoffatomen, der gegebenenfalls durch 1 bis 6 Hydroxygruppen und 1 bis 12 Fluoratome substituiert und gegebenenfalls durch 1 bis 3 Sauerstoffatome unterbrochen ist, bedeutet und
R⁶ Wasserstoff, eine gerad- oder verzweigtkettige, gegebenenfalls durch 1 bis 6 Hydroxygruppen oder 1 bis 12 Fluoratome substituierte Alkylgruppe mit 1 bis 16 Kohlenstoffatomen
oder einen Rest 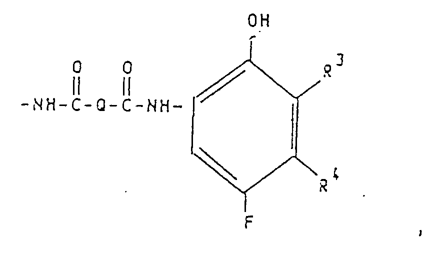 worin Q für eine (CF₂)ₖ- oder C₆F₄-Gruppe mit k in der Bedeutung der Ziffern 1, 2, 3, 4, 5 oder 6 steht,
R³ und R⁴ unabhängig voneinander Wasserstoff, Fluor, eine gerad- oder verzweigtkettige Alkylgruppe mit 1 bis 10 Kohlenstoffatomen, die gegebenenfalls durch 1 bis 6 Fluoratome substituiert ist, den Rest -N(R⁶)-CO-R⁸ oder den Rest V bedeuten,
worin V für Wasserstoff oder die Reste U-OR⁵ oder 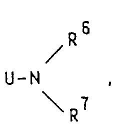 wobei
U einen CO- oder SO₂-Rest,
R⁵ Wasserstoff, einen gesättigten, ungesättigten, gerad- oder verzweigtkettigen oder cyclischen, gegebenenfalls durch 1 bis 6 Hydroxygruppen substituierten Kohlenwasserstoffrest mit bis zu 16 Kohlenstoffatomen und
R⁷ die für R⁶ angegebene Bedeutung hat oder gemeinsam mit R⁶ unter Einbeziehung des Stickstoffatoms einen gegebenenfalls ein weiteres Heteroatom enthaltenden gesättigten Fünf- oder Sechsring bilden,
mit der Maßgabe, daß mindestens 2 Fluoratome im Molekül vorhanden sind, die im Molekül vorhandenen Reste V gleich oder verschieden sein können, und gewünschtenfalls freie Säuregruppen mit organischen oder anorganischen Basen versalzt sind,

2. Diagnostische Mittel, enthaltend mindestens eine Verbindung der allgemeinen Formel I.

3. Verwendung von Verbindungen der allgemeinen Formel I als NMR-Diagnostika.

4. Verfahren zur Herstellung von fluorsubstituierten Benzolderivaten der allgemeinen Formel I 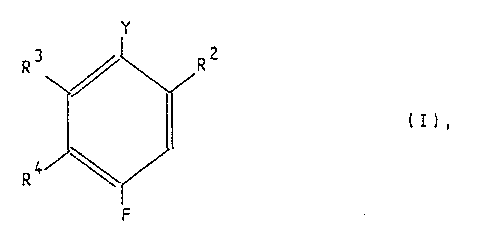 dadurch gekennzeichnet, daß man in an sich bekannter Weise in Verbindungen der allgemeinen Formel II 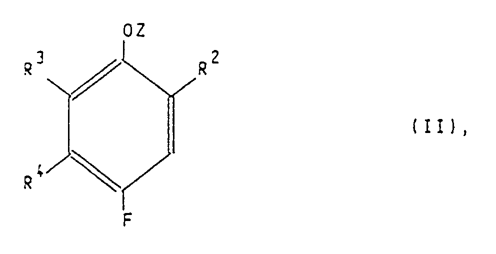 worin
Z für eine Hydroxyschutzgruppe steht, die Schutzgruppe Z abspaltet und gegebenenfalls in R³ und R⁴ vorhandene Schutzgruppen entfernt und gewünschtenfalls vorhandene freie Säuregruppen mit organischen oder anorganischen Basen versalzt.

5. Verfahren zur Herstellung der diagnostischen Mittel gemäß Anspruch 2, dadurch gekennzeichnet, daß man die in Wasser oder physiologischer Salzlösung gelöste oder suspendierte Benzolverbindung, gegebenenfalls mit den in der Galenik üblichen Zusätzen in eine für die enterale oder parenterale Applikation geeignete Form bringt.
